# EUROPEAN PATENT APPLICATION

(11) **EP 1 808 173 A1**
(43) Date of publication of application: **18.07.2007**
(21) Application number: 06000654.1
(22) Date of filing: 12.01.2006
(51) Int. Cl.: A61K 31/4188, A61K 31/4164, A61K 31/17, A61P 33/02

(54) **Use of CNS penetrating anticancer compounds for the treatment of protozan diseases**

(71) Applicant: Dormeyer, Matthias, 80637 München (DE)
(72) Inventor: Dormeyer, Matthias, 80637 München (DE)
(74) Representative: Schnappauf, Georg

(57) **Abstract**

The present invention relates to the treatment of protozoal diseases by administering cytotoxic and/or cytostatic compounds, in particular those used in anticancer therapy, to patients. In particular, the invention relates to the use of anticancer agents that can penetrate into the CNS for treatment of late stage African sleeping sickness or cerebral malaria.

## Description

### Field of the Invention

The present invention relates to the treatment of protozoal diseases by administering cytotoxic and/or cytostatic compounds, in particular those used in anticancer therapy, to patients. In particular, the invention relates to the use of anticancer agents that can penetrate into the CNS for treatment of late stage African sleeping sickness or cerebral malaria.

### Background of the Invention

African sleeping sickness (also called African trypanomiasis) is a severe disorder caused by the protozoan parasites *Trypanosoma brucei gambiense* and *T. brucei rhodesiense.* This vector-borne disease occurs only in Sub-Saharan Africa where the vector, tsetse flies (Glossina), are endemic. Interestingly there are many regions where tsetse flies are found but no cases of sleeping sickness at all. Both trypanosoma subspecies cause different forms of the disease: *T. brucei gambiense* infection leads to the Gambian (West African, chronic) form characterized by low parasitemia, gradual onset of neurological symptoms and deaths more than two years after infection. On the other hand the Rhodesian form is caused by the more virulent *T. brucei rhodesiense.* Its characteristics are a high parasitemia, a rapid onset of neurological symptoms and death often occurring only weeks after initial infection. In general disease is caused by the bite of an infected tsetse fly, but there are also other ways of transmission as mother to child infections.
Several weeks to a few months after infection the trypanosomes can invade into the central nervous system. The affected patients suffer increasingly from confusion, convulsions, apathy and weight loss. The final stage of the disease is characterized by coma. Independent of both forms, Gambian and Rhodesian, the cerebral phase is nearly exclusively fatal if untreated.

In general, existing treatments of African trypanomiasis are unsatisfactory. Only four compounds are available: suramine, pentamidine, melarsoprol and eflornithine. Suramine and pentamidine are only effective in the initial phase of the disease when the trypanosomes have not yet invaded into the CNS. Whereas suramine is used against *T. brucei rhodesiense,* pentamidine and eflornithine are administered in *T. brucei gambiense* infections. In fact, eflornithine is not effective against the Rhodesian form of sleeping sickness. Melarsoprol is presently the only available drug that can be used against the advanced stage of the Rhodesian form of African trypanomiasis. The use of this organoarsenical compound is hampered by severe side effects. An often fatal reactive encephalopathy is observed in up to 10% of all treated patients. Patients surviving these complications often suffer from serious neurological sequelae. Furthermore, the use of melarsoprol is limited by emerging resistance.

In cancer treatment several cytotoxic and cytostatic compounds have been developed to treat brain tumors (as gliomas) or brain metastases. In glioma therapy a standard therapeutic is temozolomide a prodrug that in physiological solution spontaneously converts to its active metabolite MTIC. Interestingly, this metabolite is also formed by the cytochrome P450 mediated conversion of dacarbazine (DTIC), a compound used in melanoma treatment.

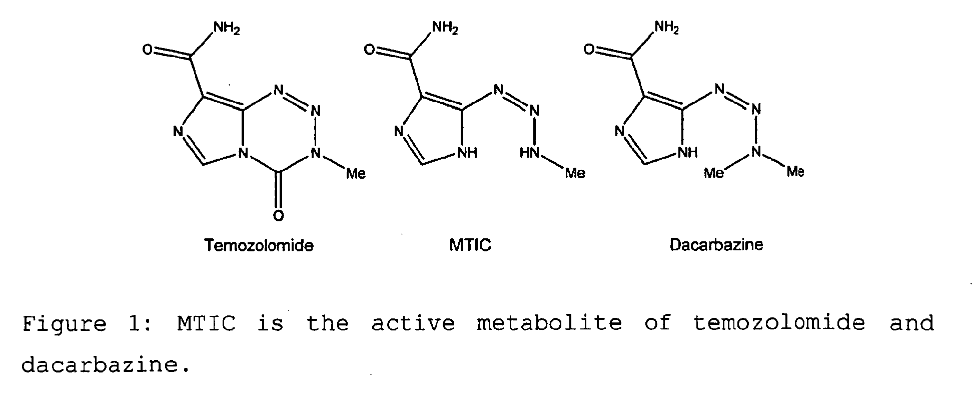

In general, it is frequently observed that drug therapy of disorders within the CNS is hampered by export systems, especially the P-glycoprotein (Pgp), encoded by the MDR1 gene. This transporter pumps neutral or cationic xenobiotics out of those cells where it is expressed in the plasma membrane. This export can be essentially complete. For instance the lack of neurological complications by loperamide is due to P-glycoprotein activity. This opioid antagonist enters the CNS but the exposition of the brain is negligible due to the high efflux mediated by this transporter (Schinkel et al. (1996): J. Clin. Invest. 97(11), 2517 - 2524).
P-glycoprotein plays also an important role in the development of multi-drug resistance (MDR) of malignant cells. The phenotype of MDR is frequently associated with increased expression of P-glycoprotein. As in the CNS Pgp pumps the drug molecules out of the cell and the tissue thus abolishing their pharmacological action.
Typically the P-glycoprotein transports neutral and cationic organic compounds (Gottesman (2002): Annu. Rev. Med. 53, 615 - 627). In cancer therapy many compounds have been identified as substrates of Pgp, for instance docetaxel, paclitaxel, vinblastine and daunorubicine.

Numerous inhibitors of Pgp have been identified in the past and many of them have been tested in clinical trials. These trials typically aimed at increasing the potency of cytotoxic anticancer agents against resistant tumors or other malignant cells. Examples for Pgp inhibitors are cyclosporine A, valpodar, elacridar, tariquidar, zosuquidar, laniquidar, biricodar, S-9788, MS-209, BIBW-22 (BIBW-22-BS), toremifene, verapamil, dexverapamil, quinine, quinidine, *trans-*flupentixol, chinchonine and others (J. Roberts, C. Jarry (2003): J. Med. Chem. 46, 4805 - 4817). The list of inhibitors of P-glycoprotein is increasing (e.g. Wang et al. (2002): Bioorg. Med. Chem. Lett. 12, 571 - 574).

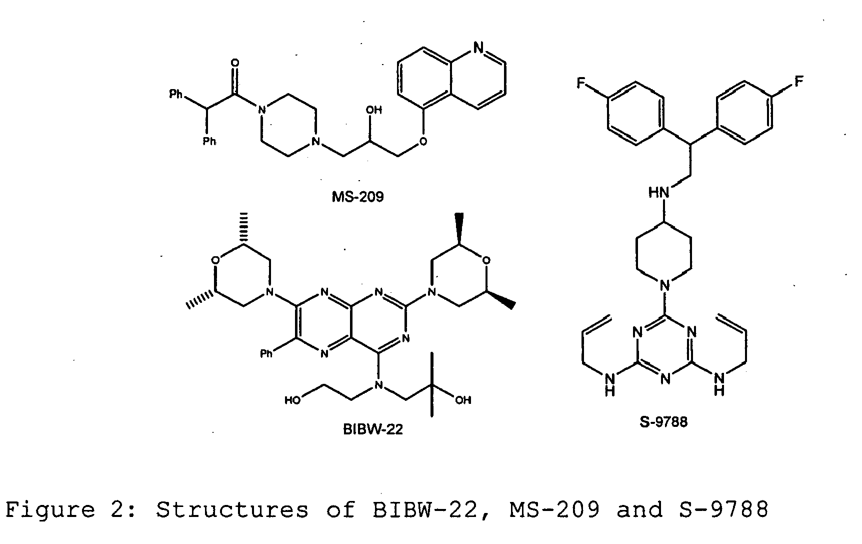

The use of inhibitors of P-glycoprotein typically aims at the treatment of multidrug resistant cancers where the resistance is confessed by overexpression of P-glycoprotein.

It was the object of the present invention to provide methods for a therapy of protozoal diseases. This object is achieved by the subject-matter of the claims.

### Description of the Invention

The present invention relates to a method for the treatment of a protozoal disease in a patient comprising the step of administering at least one cytotoxic and/or cytostatic compound or a prodrug thereof, or combinations of these compounds to the patient. Preferably, the cytotoxic and/or cytostatic compound or the prodrug thereof is one which has been used and is preferably effective in anticancer therapy, preferably in the treatment of brain tumors or brain metastases. Preferably, the cytotoxic and/or cytostatic compound or the prodrug thereof has the ability to penetrate into the CNS of a patient.

In a further preferred embodiment of the method of the invention, the protozoal disease is African sleeping sickness or cerebral malaria.

In one aspect of the method of the invention, the cytotoxic and/or cytostatic compound and/or the prodrug thereof is not a substrate of P-glycoprotein.

Preferred embodiments of cytotoxic and/or cytostatic compounds according to this aspect are those of the general formula (I) or prodrugs thereof: 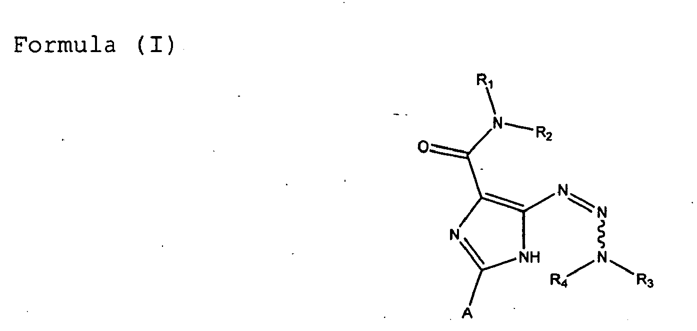 wherein
R₁, R₂, R₃ and R₄ are independently selected from the group consisting of hydrogen, alkyl, cycloalkyl, aryl, and heteroaryl which alkyl, cycloalkyl, aryl, and heteroaryl are optionally substituted at one or more positions with substituents independently selected from the group consisting of halogen, hydroxy, amino, alkoxy, alkylamino, haloalkyl, and haloalkoxy and
A is selected from the group consisting of hydrogen, alkyl and haloalkyl.

Prodrugs of the compounds of formula (I) include but are not limited to compounds according to the general formula (II): 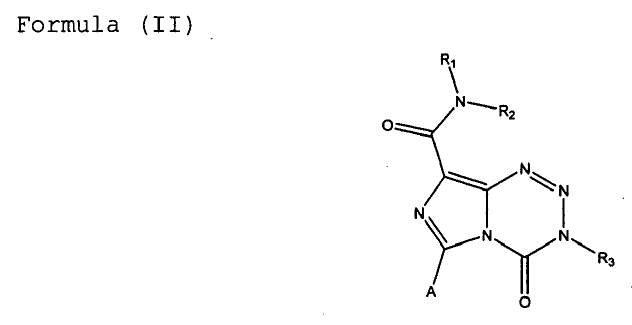 wherein
R₁, R₂ and R₃ are independently selected from the group consisting of hydrogen, alkyl, cycloalkyl, aryl, and heteroaryl which alkyl, cycloalkyl, aryl, and heteroaryl are optionally substituted at one or more positions with substituents independently selected from the group consisting of halogen, hydroxy, amino, alkoxy, alkylamino, haloalkyl, and haloalkoxy and
A is selected from the group consisting of hydrogen, alkyl or haloalkyl.

Preferred compounds of the general formula (I) are MTIC or dacarbazine, and a preferred prodrug of the general formula (II) is temozolomide.

Further embodiments of cytotoxic and/or cytostatic compounds for use in the method of the invention include busulfan and treosulfan.

Further preferred embodiments of cytotoxic and/or cytostatic compounds for use in the method of the invention are those of the general formula (III): 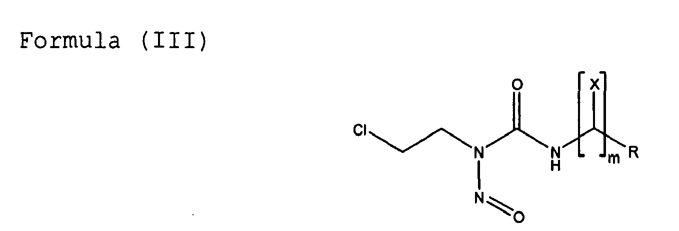 wherein
R is selected from the group consisting of halogen, alkyl, cycloalkyl, aryl, heteroary which alkyl, cycloalkyl, aryl, and heteroaryl are optionally substituted at one or more positions with substituents selected from the group consisting of halogen, alkyl, cycloalkyl, haloalkyl, amino, alkylamino, hydroxy, alkoxy, haloalkoxy, aryl and heteroaryl, and
X is selected from the group consisting of hydrogen, alkyl, aryl, halogen, heteroaryl, phosphonic acid ester, hydroxy, amino, alkylamino, acylamino, alkoxy, haloalkoxy and C(O)OR', wherein R' is alkyl or haloalkyl, and
m is 0 or 1.

Preferred compounds of the general formula (III) are selected from the group consisting of carmustine (BCNU), fotemustine, lomustine (CCNU), and nimustine (ACNU).

In a further aspect of the method of the invention, the cytotoxic and/or cytostatic compound and/or the prodrug thereof is a substrate of P-glycoprotein. According to this aspect of the invention, especially in cases where the exposition of the central nervous system to the compound is not sufficiently high due to the fact that the compound is exported via the action of the Pgp transporter, it is preferred to co-administer at least one inhibitor of P-glycoprotein.

Preferred cytotoxic and/or cytostatic compounds according to this aspect of the invention are taxanes, more preferably docetaxel and paclitaxel, or structural derivatives thereof.

Further preferred embodiments of cytotoxic or cytostatic compounds according to this aspect of the invention are those having a structure that is characterized by at least four fused cyclic moieties selected from cyclic aromatic or aliphatic moieties optionally containing heteroatoms such as oxygen, sulfur or nitrogen. Examples for this kind of compounds are vinca alkaloids or anthracyclines.

Preferred anthracyclines are doxorubicin, daunorubicine, idarubicine, epirubicine or any of their prodrugs.

Preferred vinca alkaloids are eburnamonine, vinblastine, vindesine, vincristine or vinorelbine alkaloids or any of their prodrugs.

Inhibitors of Pgp for use in the method according to the invention include but are not limited to compounds according to the general formulas (IV), (V), and (VI): 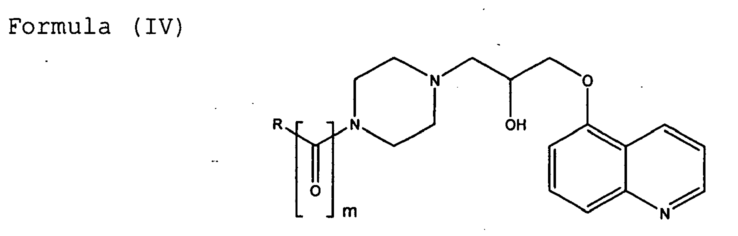 wherein
m is 0 or 1, and
R is selected from the group consisting of aryl, heteroaryl, cycloalkyl optionally containing 1 or 2 heteroatoms, and 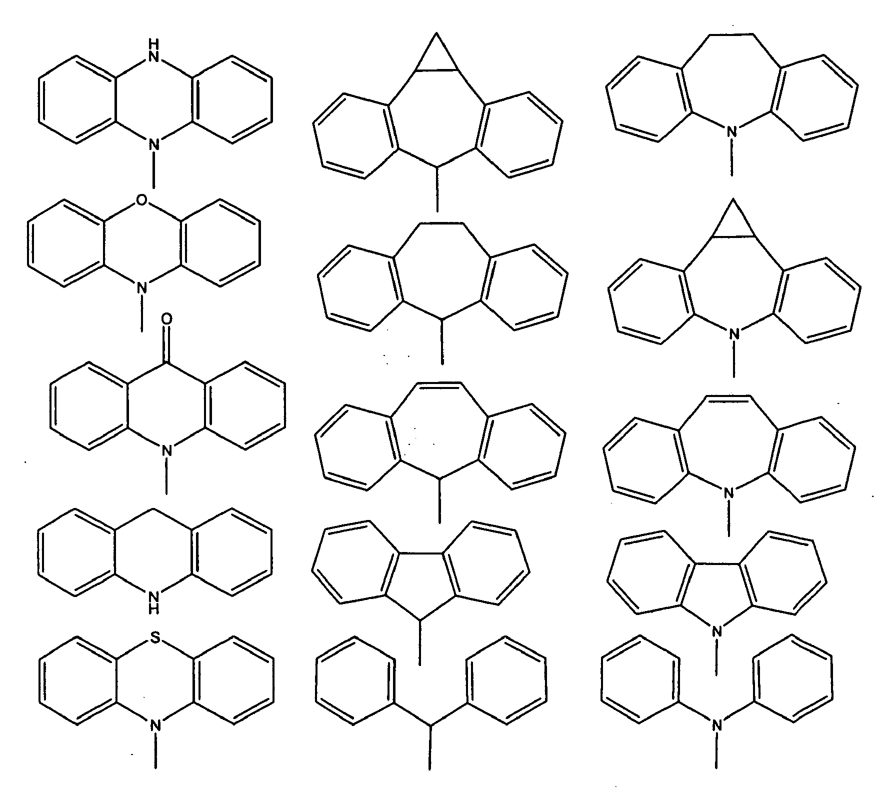 which groups are optionally substituted at one or more positions with substituents selected from the group consisting of halogen, alkyl, haloalkyl, hydroxy, alkoxy, haloalkoxy, amino, alkylamino, carboxyl, and acylamino.

Preferred compounds according to the general formula IV are zosuquidar (LY-335979) and MS-209. 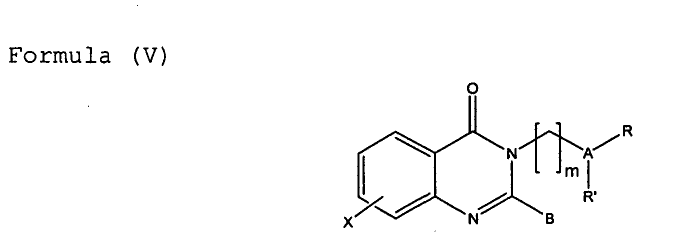 wherein
X is selected from the group consisting of hydrogen, halogen, alkyl, haloalkyl, hydroxy, alkoxy, haloalkoxy, amino, and alkylamino,
m is 0, 1 or 2,
A is selected from the group consisting of aryl, heteroaryl, cycloalkyl which aryl, heteroaryl and cycloalkyl are optionally substituted at one or more positions with substituents selected from the group consisting of halogen, alkyl, amino, alkylamino, hydroxy, alkoxy, haloaryl, and haloalkoxy,
B is a carbocyclic or heterocyclic, aromatic or nonaromatic moiety optionally substituted at one or more positions with substituents selected from the group consisting of halogen, alkyl, haloalkyl, hydroxy, alkoxy, haloalkoxy, amino, and alkylamino,
R and R' are independently selected from the group consisting of hydrogen, alkyl, alkoxy, aryl, heteroaryl, benzyl, benzoyl, alkoxy, haloalkoxy, halogen, and hydroxy. 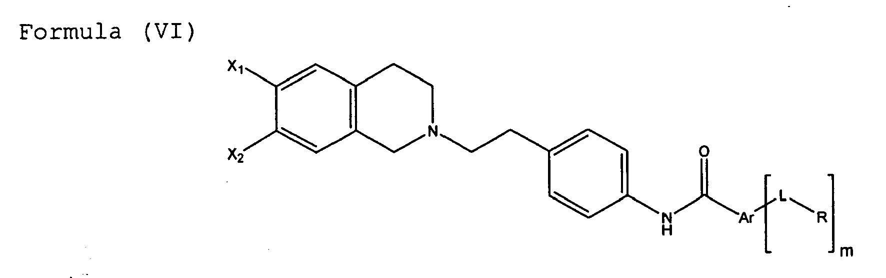
wherein
X₁ and X₂ are independently selected from the group consisting of hydrogen, halogen, alkyl, haloalkyl, hydroxy, alkoxy, haloalkoxy, amino, and alkylamino,
Ar is aryl, heteroaryl or 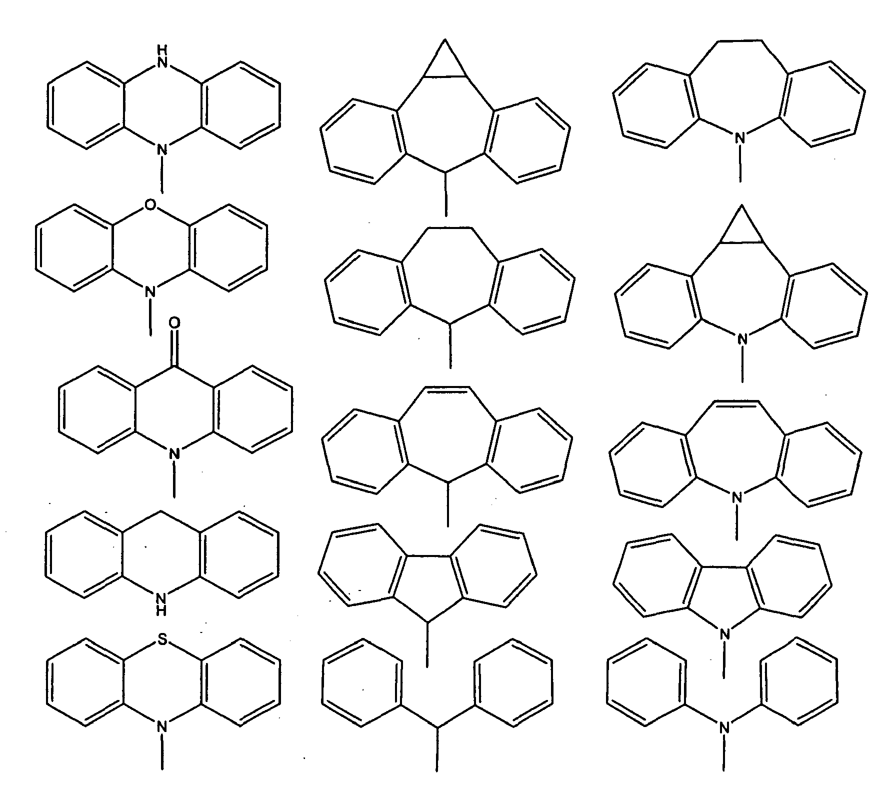 optionally substituted at one or more positions with substituents selected from the group consisting of halogen, alkyl, haloalkyl, hydroxy, alkoxy, haloalkoxy, amino, and alkylamino,
L is selected from the group consisting of: 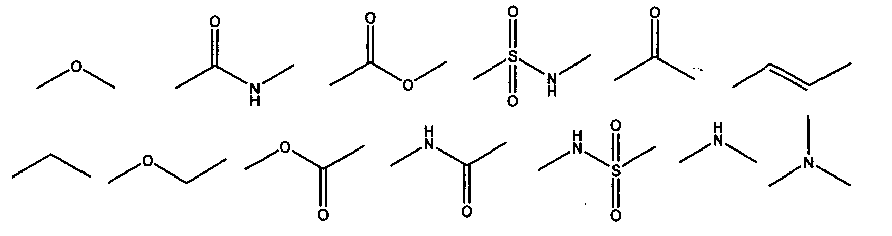
R is selected from the group consisting of alkyl, aryl, and heteroaryl, and
m is 0 or 1.

Preferred compounds according to the general formula VI are tariquidar (XR-9576) and elacridar (GF-120918).

Further embodiments of inhibitors of P-glycoprotein for use in the method of the invention comprise cyclic peptides or modified cyclic peptides, preferably consisting of 8 - 12 amino acids. Particularly preferred embodiments are cyclosporin A or valspodar (PSC-833).

Further specific embodiments of inhibitors of P-glycoprotein are selected from the group consisting of verapamil, dexverapamil, nifedipine, dexniguldipine, amiodarone, bepridil, cinchonine, quinine, quinidine, tamoxifen, toremifene, progesterone, dipyridamole, trifluoperazine, *trans*-flupentixol, biricodar (VX-710), S-9788, BIBW-22, and laniquidar (R-101933). Particularly preferred inhibitors of P-glycoprotein for use in the method of the invention are cinchonine, toremifene, trans-flupentixol, biricodar (VX-710), S-9788, laniquidar (R-101933), zosuquidar (LY-335979), MS-209, tariquidar (XR-9576), BIBW-22 elacridar (GF-120918), cyclosporin A or valspodar (PS-833).
Most preferred inhibitors of P-glycoprotein are biricodar (VX-710), laniquidar (R-101933), zosuquidar (LY-335979), MS-209, tariquidar (XR-9576) elacridar (GF-120918) or valspodar (PS-833).

In a preferred embodiment of the method of the invention, one or more cytotoxic and/or cytostatic compounds selected from the group consisting of paclitaxel, docetaxel, eburnamonine, vinblastine, vindesine, vincristine, vinorelbine, doxorubicin, daunorubicine, idarubicine, and epirubicine are administered in combination with one or more inhibitors of P-glycoprotein selected from the group consisting of cinchonine, toremifene, trans-flupentixol, biricodar (VX-710), S-9788, BIBW-22, laniquidar (R-101933), zosuquidar (LY-335979), MS-209, tariquidar (XR-9576), elacridar (GF-120918), cyclosporin A and valspodar. More preferably, the one or more cytotoxic and/or cytostatic compounds are selected from the group consisting of doxorubicin, vinblastine, paclitaxel or docetaxel and the one or more inhibitors of P-glycoprotein are selected from the group consisting of vaspodar (PS-833), biricodar (VX-710), elacridar (GF-120918), trans-flupentixol, zosuquidar (LY-335979), tariquidar (XR-9576) or laniquidar (R-101933). Preferably, the protozoal disease which is treated by said combination is African sleeping sickness or cerebral malaria, more preferably the cerebral phase of African sleeping sickness or cerebral malaria.

The term "alkyl" refers to a monoradical branched or unbranched saturated or unsaturated hydrocarbon chain having from 1 to 20 carbon atoms. This term is exemplified by groups such as methyl, ethyl, n- propyl, iso-propyl, n-butyl, tert-butyl, n-hexyl, n-decyl, tetradecyl, and the like.
Cycloalkyl groups are monocyclic, bicyclic or tricyclic ring systems of 3-8, more preferably 3-6, ring members per ring, such as cyclopropyl, cyclobutyl, cyclopentyl, cyclohexyl, adamantyl and the like. Alkyl also includes a straight chain or branched alkyl group that contains or is interrupted by a cycloalkyl portion. The straight chain or branched alkyl group is attached at any available point to produce a stable compound. Examples of this include, but are not limited to, 4-(isopropyl)-cyclohexylethyl or 2-methyl-cyclopropylpentyl.

The term "aryl" refers to an aromatic carbocyclic group of 6 to 20 carbon atoms having a single ring (e.g. phenyl) or multiple rings (e.g. biphenyl) or multiple condensed (fused) rings (e.g. naphthyl). Preferred aryls include phenyl, naphthyl and the like.

The term "heteroaryl" refers to an aromatic group (i.e. unsaturated) comprising 1 to 15 carbon atoms and 1 to 6 heteroatoms selected from the group consisting of oxygen, nitrogen and sulfur within at least one ring. Such heteroaryl groups can have a single ring (e.g. pyridyl or furyl) or multiple condensed rings (e.g. indolizinyl or benzothiazolyl). Examples of heteroaryls include but are not limited to pyrrole, imidazole, pyrazole, pyridine, pyrimidine, pyridazine, indolizine, isoindole, indole, indazole, purine, quinolizine, quinoline, isoquinoline, phthalazine, naphthylpyrimidine, quinoxaline, quinazoline, cinnoline, thiophene, pteridine, thiazole, benzothiazole, carbazole, carboline, phenanthridine, acridine, phenanthroline, isothiazole, phenazine, isoxazole, phenoxazine, phenothiazine, imidazolidine, imidazoline, an the like as well as N-alkoxy-nitrogen containing heteroaryl compounds.

The term "hydroxy" refers to the group -OH.

The term "halogen" refers to fluoro, bromo, chloro and iodo.

The term "haloalkyl" refers to an alkyl or cycloalkyl group substituted with one or more halogen (e.g. trifluoromethyl).

The term "alkoxy" refers to the group -OR, where R is alkyl, aryl or cycloalkyl.

The term "haloalkoxy" refers to the group -OR, where R is alkyl, aryl or cycloalkyl substituted with one or more halogen

The term amino refers to the group -NH₂.

The term "alkylamino" refers to the group -NR'R where R is hydrogen, alkyl, aryl or cycloalkyl and where R' is alkyl, aryl or cycloalkyl.

The term "acylamino" refers to the group -NRC(O)R where each R is independently hydrogen, alkyl, aryl, or heteroaryl.

The term "carbonyl" refers to the group C=O wherein the carbon can be part of an alkyl chain or ring system.

The term "phosphonic acid ester" refers to the group - P(O(OR)(OR') where R is hydrogen, alkyl, aryl or cycloalkyl and where R' is alkyl, aryl or cycloalkyl.

The modified peptides described herein may comprise any natural and non-natural modifications, in particular chemical or physical modifications. The term "natural modification" relates to any modification found in peptides or proteins in nature such as posttranslational modifications, modifications due to exposure to light, oxygen, exposure to acid or alkali solutions, etc. The term "non-natural modification" relates to non-naturally occurring modifications as found, for example, in peptidomimetics.

Examples of possible modifications include but are not limited to: glycosylation, phosphorylation, sulphatation, pyroglutamate modification, cystein-disulfide bridges, methylation, acetylation, acylation, farnesylation, formylation, geranylgeranylation, biotinylation, stearoylation, palmitylation, lipolyation, C-mannosylation, myristoylation, amidation, deamidation, methylation, demethylation, carboxylation, hydroxylation, iodination, oxidation, pegylation, prenylation, ADP-ribosylation, addition of lipids, of phosphatidylinositol, of glycosylphosphatidylinositol (GPI)-anchor, of pyridoxal phosphate, modification of cysteine residues resulting in carboxyamidomethylcysteine, resulting in carboxymethylcysteine, or resulting in pyridylethylcysteine, modification of lysine residues resulting in liponic acid, modification of glutamic acid resulting in pyroglutamic acid, etc.

Modifications of peptides described herein may comprise unusual amino acids, chemically or enzymatically modified amino acids etc. including, but not limited to: alpha amino butyric acid, beta amino butyric acid, beta amino iso-butyric acid, beta alanine, gamma butyric acid, alpha amino adipic acid, 4-amino benzoic acid, amino ethyl cysteine, alpha amino penicillanic acid, allysine, 4-carboxy glutamic acid, cystathionine, carboxy glutamic acid, carboxy amido methyl cysteine, carboxy methyl cysteine, cysteine acid, citrulline, dehydroalanine, di-amino butyric acid, dehydro amino-2-butyric acid, ethionine, glycine-proline di-peptide, 4-hydroxyproline, hydroxylysine, hydroxyproline, homoserine, homo cysteine, histamine, iso-valine, lysinoalanine, lanthionine, norvaline, norleucine, ornithine, 2-pipiridine-carboxylic acid, pyroglutamic acid, pyrrolysine, proline-hydroxy proline di-peptide, sarcosine, 4-selenocysteine, syndesine, thioproline, etc.

Peptides may further represent partially or completely peptidomimetics. Peptidomimetics can replace some or all peptide bonds by other kinds of covalent bonds which can connect amino acids. Peptidomimetics among others can comprise modifications of amino acid residues such as alpha-C alkylation, alpha-N alkylation, etc, dipeptide analogues (e.g. two amino acid side chains which are connected by covalent bonds) or modifications of the peptide backbone, especially change from L- to D-amino acid residues, inverse N- to C-sequence, amide bond isosteres, peptoides (e.g. oligomers of N-substituted glycines, PNAS, 1992, 89:9367), retro-inverso-peptidomimetica (Acc Chem Res, 1993, 26:266), oligocarbamates (peptide bond replaced by carbamate structure; Science, 1993, 261:1303), oligopryrrolinones (J Am Chem Soc, 1992,114:10672), vinylopeptides (J Am Chem Soc, 1992, 114:6570), etc.

A prodrug is a precursor of a drug. A prodrug is usually biological inactive at its site of action, but will undergo conversion spontaneously or by metabolic processes to the bioactive form. A prodrug may be a drug which has been chemically modified, wherein the modifications may be (1) ester or carbamate derivatives which may be cleaved by esterases or lipases, for example; (2) peptides which may be recognized by specific or non-specific proteinases; or (3) derivatives that accumulate at a site of action through membrane selection.

The present invention includes all structural derivatives of the specific compounds or groups of compounds disclosed herein.

The term "structural derivative" which refers to variants derived from the specific compounds disclosed herein, is used herein in a conventional chemical sense. The term "structural derivative" includes molecules which resemble another designated molecule, but which has been modified by one or more substituted or altered chemical groups. For example, in a derivative, a specific hydrogen atom or hydroxyl group attached to a molecule at a certain location might be replaced by halogen atoms, alkyl or acyl groups, or various other chemical substituents. If the resulting derivative still has the full set of desirable properties discussed herein, it may be useful for treatment of a protozoal disease, as disclosed herein. Certain derivatives may have somewhat greater potency than a reference molecule, and may therefore rise to the level of an improvement.

One class of derivatives that are of interest herein include derivatives that have been modified in ways designed to render them more capable of permeating through the blood-brain barrier (BBB). The electrochemical traits of molecules that either can or cannot permeate through mammalian BBB's are reasonably well understood, and strategies have been developed for modifying drug molecules in specific limited ways that are designed to increase their ability to permeate through BBB's.

Various derivatives of the specific compounds listed herein can be synthesized, using standard chemical synthesis procedures, and tested for their ability to penetrate into the CNS and efficacy in treating a protozoal disease.

"Optional" or "optionally" means that the subsequently described event or circumstance may or may not occur, and that the description includes instances where said event or circumstance occurs and instances in which it does not.

According to the invention, the compounds listed above also include all pharmacological acceptable salts of said compounds, including but not limited to those where the counterions are selected from the group consisting of acetate, 2,2-dichloroacetate, adipate, alginate, ascorbate, aspartate, benzoate, benzenesulfonate, 2-acetamidobenzoate, caproate, caprate, camphorate, camphorsulfonate, cinnamate, citrate, cyclamate, laurylsulfate, edisilate, esylate, isetionate, formate, fumarate, galactarate, gentisate, gluceptate, gluconate, glucuronate, glutamate, oxoglutarate, glycolate, hippurate, bromide or hydrobromide, chloride or hydrochloride, lactate, lactobionate, malate, maleate, malonate, mandelate, mesylate, napsilate, napadisilate, xinafoate, nicotinate, nitrate, oleate, orotate, oxalate, palmitate, embonate, phosphate or hydrogenphosphate, pidolate, salicilate, p-aminosalicylate, sebacate, stearate, succinate, sulfate or hydrogensulfate, tannate, tartrate, rhodanide, tosylate, undecylenate, ammonium, arginine, benethamine, benzathine, calcium, choline, deanol, diethanolamine, diethylammonium, ethanolamine, ethylendiamine, meglumine, hydrabamine, imidazole, lysine, magnesium, hydroxyethylmorpholine, piperazine, potassium, epolamine, sodium, trolamine, tromethamine or zinc.

The compounds described herein may be administered via any conventional route. The administration may be carried out, for example, orally, intravenously, intraperitonealy, intramuscularly, subcutaneously or transdermally.

The compounds described herein are administered in effective amounts. An "effective amount" refers to the amount which achieves a desired reaction or a desired effect alone or together with further doses. In the case of treatment of a particular disease or of a particular condition, the desired reaction relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition.

The doses administered of the compounds described herein may depend on various parameters such as the type of administration, the condition of the patient, the desired period of administration, etc. In the case that a reaction in a patient is insufficient with an initial dose, higher doses (or effectively higher doses achieved by a different, more localized route of administration) may be used.

Preferably, the compounds described herein are administered in doses of 0.1 to 10000 mg per day. Preferred daily dose for particular groups of compounds comprise the following ranges: anthracyclines: 1 to 1000 mg, nitrosourea derivatives: 4 to 1800 mg, MTIC like compounds and their products: 2.5 to 7500 mg, taxanes:2 to 2000 mg, vinca alkaloids: 0.1 to 250 mg, Pgp inhibitors:1 to 10000 mg. More preferred daily doses for particular compounds comprise the following ranges: BIBW-22: 5 to 250 mg, biricodar: 100 to 150 mg/m², busulfan: 6- 600 mg/m², carmustine: 100 to 200 mg/m², dexverapamil: 120 to 500 mg, chinidinsulfate: 200 to 1000 mg, chininesulfate: 200 to 250 mg, cinchonine: 15 to 35 mg, cyclosporine A: 2 to 5 mg/kg, dacarbazine: 200 to 850 mg/m², daunorubicine: 3 to 500 mg; 40. to 60 mg/m², dexniguldipine: 100 to 2500 mg, dipyridamole: 200 to 400 mg, docetaxel: 40 to 100 mg/m², elacridar: 100 to 1000 mg, epirubicine: 40 to 120 mg/m², fotemustine: 50 to 200 mg/m², idarubicine: 15 to 30 mg/m², laniquidar: 100 to 750 mg, lomustine: 50 to 100 mg/kg, MS-209: 300 to 1200 mg, nifedipine: 15 to 60 mg, nimustine: 60 to 100 mg/m², paclitaxel: 75 to 225 mg/m², S9788: 80 to 100mg, tariquidar: 100 to 750 mg, temozolomide: 50 to 250 mg/m², treosulfan: 1000 to 30000 mg, trifluoperazine: 20 to 75 mg, valspodar: 2 to 15 mg/kg, verapamil: 100 to 500 mg, vinblastinesulfat: 0.5 to 7.5 mg/m², vincristinesulfat: 0.5 to 2.5 mg/m², vindesine: 1.5 to 6 mg, vinorelbine: 10 to 25 mg/m², zosuquidar: 100 to 640 mg/m².

The terms "protozoal disease" or "protozoal infection" refer to any disease, disorder or infection caused by pathogenic parasites of the phylae Apicomplexa (Sporozoa) or Sarcomastigophora, especially caused by parasites of the species *Plasmodium falciparum, Plasmodium ovale, Plasmodium vivax, Plasmodium malariae, Toxoplasma gondii, Leishmania major, Leishmania infantum, Leishmania tropica, Leishmania mexicana, Leishmania donovanii, Leishmania brasilensis, Trypanosoma cruzi, Trypanosoma brucei rhodesiense, Trypanosoma brucei gambiense, Isospora belli, Sarcocystis suihominis, Cryptosporidium parvum* or *Entamoeba histolytica.* Preferably, the term "protozoal disease" refers to the cerebral phase of a protozoal disease.

African sleeping sickness (also called African trypanosomiasis) is caused by the protozoal species *T. brucei rhodesiense* and *T. brucei gambiense.*

Cerebral malaria is a complication of the malaria tropica, caused by *Plasmodium falciparum* that is characterized by changes in mental status and coma.

According to the invention, the term "cytostatic compound" refers to any agent with the ability to inhibit or suppress growth or multiplication of eukaryotic cells, or a prodrug thereof.

According to the invention, the term "cytotoxic compound" refers to any agent relating to or producing a toxic effect to eukaryotic cells, or a prodrug thereof. Toxic effects include but are not limited to apoptosis and necrosis.

The compounds described herein are generally administered in pharmaceutically compatible compositions. The term "pharmaceutically compatible" refers to a nontoxic material which does not interact with the action of the active component of the pharmaceutical composition. Preparations of this kind may usually contain salts, buffer substances, preservatives, carriers and, where appropriate, other therapeutically active compounds. When used in medicine, the salts should be pharmaceutically compatible. However, salts which are not pharmaceutically compatible may used for preparing pharmaceutically compatible salts and are included in the invention.

A pharmaceutical composition of the invention may comprise a pharmaceutically compatible carrier. According to the invention, the term "pharmaceutically compatible carrier" refers to one or more compatible solid or liquid fillers, diluents or encapsulating substances, which are suitable for administration. The term "carrier" refers to an organic or inorganic component, of a natural or synthetic nature, in which the active component is combined in order to facilitate application. The components of the pharmaceutical composition of the invention are usually such that no interaction occurs which substantially impairs the desired pharmaceutical efficacy.

The pharmaceutical compositions of the invention may contain suitable buffer substances such as acetic acid in a salt, citric acid in a salt, boric acid in a salt and phosphoric acid in a salt.

The pharmaceutical compositions may, where appropriate, also contain suitable preservatives such as benzalkonium chloride, chlorobutanol, paraben and thimerosal.

The pharmaceutical compositions are usually provided in a uniform dosage form and may be prepared in a manner known per se. Pharmaceutical compositions of the invention maybe in the form of capsules, tablets, lozenges, solutions, suspensions, syrups, elixirs or in the form of an emulsion, for example.

Compositions suitable for parenteral administration usually comprise a sterile aqueous or nonaqueous preparation of the active compound, which is preferably isotonic to the blood of the recipient. Examples of compatible carriers and solvents are Ringer solution and isotonic sodium chloride solution. In addition, usually sterile, fixed oils are used as solution or suspension medium.

### Examples

### Compound availability

All compounds mentioned in the example section can be purchased from commercial providers, e.g., Sigma or Sequoia Research.

### Sleeping sickness: In vitro screening model

### Parasite cultures

Three strains of *Trypanosoma brucei spp.* are used in this study: (a) *T. b. rhodesiense STIB* 900 (a clone of a population isolated in 1982 from a patient in Tanzania), which is known to be susceptible to all currently used drugs; (b) T. b. gambiense STIB 930 (a derivative of strain TH1/78E (031), isolated in 1978 from a patient in Ivory Coast), which is known to be sensitive to all drugs used; and (c) T. b. brucei STIB 950 (a clone of a population isolated in 1985 from a bovine in Somalia), which shows drug resistance to diminazene, isometamidium and quinapyramine.
Bloodstream form trypomastigotes of the strains (a) and (c) are maintained in Minimal Essential Medium (MEM) with Earle's salts supplemented according to Baltz et al. (EMBO J. 4, 1273-1277,1985) with 25 mM N-2-hydroxyethylpiperazine-N'-2-ethanesulphonic acid (HEPES), 1 g/l additional glucose, 1% MEM non-essential amino acids (100x), 0.2 mM 2-mercaptoethanol, 2 mM sodium pyruvate, 0.1 mM hypoxanthine and 15% heat-inactivated horse serum.
Bloodstream form trypomastigotes of strain (b) are maintained in MEM with Earle's salts supplemented with 25 mM HEPES, 1 g/l additional glucose, 1% MEM non-essential amino acids (100x), 0.2 mM 2-mercaptoethanol, 2 mM sodium pyruvate, 0.1 mM hypoxanthine, 0.05 mM bathocuproine disulphonic acid, 0.15 mM L-cysteine and 15% heat-inactivated pooled human serum.
All cultures and assays are conducted at 37°C under an atmosphere of 5% CO₂ in air.

### Drug sensitivity assays

Stock drug solutions are prepared in 100% dimethylsulphoxide (DMSO) (unless otherwise recommended by the supplier) at 10 mg/ml, and heated or sonicated if necessary to dissolve the sample. The stocks are kept at -20°C. For the assays, the compound is further diluted to the appropriate concentration using complete medium. The DMSO concentration in the wells with the highest drug concentration does not exceed 1%.

Assays are performed in 96-well microtiter plates, each well containing 100 µl of culture medium with 8 x 10³ bloodstream forms with or without a serial drug dilution. The highest concentration of the test compounds is 90 µg/ml. Seven 3-fold drug dilutions are used, covering a range from 90 µg/ml to 0.123 µg/ml. Each drug is tested in duplicate. Active compounds are tested twice for confirmation. The final result is the mean of the four individual IC₅₀ values. After 72 hrs of incubation, the plates are inspected under an inverted microscope to assure growth of the controls and sterile conditions.

Ten microliters of Alamar Blue (12.5 mg resazurin dissolved in 100 ml distilled water) are then added to each well and the plates are incubated for another 2 hours. The plates are read with a Spectramax Gemini XS microplate fluorometer (Molecular Devices Cooperation, Sunnyvale, CA, USA) using an excitation wave length of 536 nm and an emission wave length of 588 nm.

IC₅₀ values are determined using the microplate reader software Softmax Pro (Molecular Devices Cooperation, Sunnyvale, CA, USA).

### Results:

**Table 1: Activity of various test compounds against Trypanosoma brucei rhodesiense (STIB 900 clone) in vitro:**

| **Compound** | **IC₅₀ [µg/ml]** |
|---|---|
| Docetaxel | 0.006 |
| Paclitaxel | 0.010 |
| Doxorubicin | 0.015 |
| Estramustine | 41.5 |
| Vinblastine | 0.13 |
| Cyclosporin A | 7.57 |
| Temozolomide | 27.7 |
| Dacarbazine | 19,7 |
| Carmustine | 2.18 |

### Sleeping sickness: In vivo chronic CNS model -Trypanosoma b. brucei GVR 35

### Parasite and animal strains

*Trypanosoma brucei brucei* GVR 35 which leads to a CNS infection by day 21 post-infection (Jennings and Gray, Contrib. Microbiol. Immunol. 7,147-54,1983) is used for this model.
Female mice (NMRI), weighing 20-25 g, are maintained at 22°C and 70% relative humidity.

### General experimental procedure

Infection: The bloodstream forms are from a stock of cryopreserved stabilates containing 10% glycerol. The stabilate is suspended in PSG (phosphate-saline-glucose) 6:4 (Lanham & Godfrey, Exp. Parasitol. 28, 521-534, 1970) to obtain a trypanosome concentration of 8x10⁴/ml. Each mouse is intraperitoneally injected with 0.25 ml inoculum (equivalent to 2x10⁴ trypanosomes) on day 0.

Experimental groups and control groups each consist of 5 mice. Evaluation endpoint: On day +28 and then twice a week until day +50 parasitaemia of all animals is checked by tail blood examination and recorded based on a subjective scale ranging from (+) = 1 trypanosome/20 fields to +++ = >100 trypanosomes/field with a 20x objective. After day +50 the mice are checked only once per week until day +180. For mice dying before day +180 the day of death is recorded.
At day +180, surviving and aparasitaemic mice are considered cured and then euthanized.

### Treatment regimen:

Compounds are prepared at appropriate concentrations in 100% DMSO and further diluted in distilled water, unless another solvent is recommended by the supplier. The total amount of solvent administered has to be below toxicity (for DMSO i.p. <10%).
Test items are daily administered i.p. or p.o. in a total volume of 10 ml/kg body weight from day +21 to day +25.
Diminazene diaceturate at 40 mg/kg given as single i.p. dose on day +21 serves as a negative control, i.e. all mice clear parasitaemia but relapse before day +40 and die between day +50 and day +60.

## Claims

1. Use of at least one cytotoxic and/or cytostatic compound or a prodrug thereof for the preparation of a pharmaceutical composition for the treatment of a protozoal disease in a patient.

2. The use according to claim 1 wherein the protozoal disease is **characterized by** cerebral complications.

3. The use according to claim 1 or 2 wherein the protozoal disease is African sleeping sickness or cerebral malaria.

4. The use according to any one of claims 1 to 3 wherein the cytotoxic and/or cytostatic compound or the prodrug thereof has the ability to penetrate into the CNS.

5. The use according to any one of claims 1 to 4 wherein the cytotoxic and/or cytostatic compound or the prodrug thereof is not a substrate of P-glycoprotein.

6. The use according to any one of claims 1 to 5 wherein the cytotoxic and/or cytostatic compound is an alkylating agent.

7. The use according to any one of claims 1 to 6 wherein the cytotoxic and/or cytostatic compound is a compound of the general formula (I): 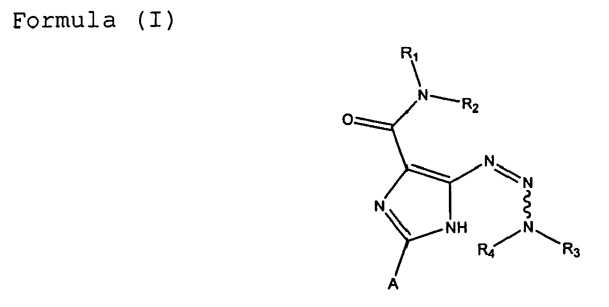 wherein
R₁, R₂, R₃ and R₄ are independently selected from the group consisting of hydrogen, alkyl, cycloalkyl, aryl, and heteroaryl which alkyl, cycloalkyl, aryl, and heteroaryl are optionally substituted at one or more positions with substituents independently selected from the group consisting of halogen, hydroxy, amino, alkoxy, alkylamino, haloalkyl, and haloalkoxy and
A is selected from the group consisting of hydrogen, alkyl and haloalkyl.

8. The use according to claim 7 wherein the prodrug has the general formula (II) 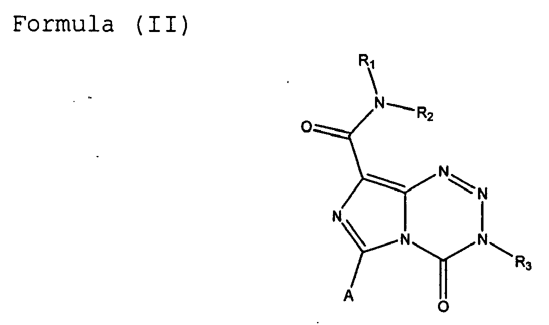 wherein
R₁, R₂, R₃, and A are as defined in claim 7.

9. The use according to any one of claims 1 to 8 wherein the cytotoxic and/or cytostatic compound or the prodrug thereof is selected from the group consisting of MTIC, temozolomide or dacarbazine.

10. The use according to any one of claims 1 to 6 wherein the cytotoxic and/or cytostatic compound is a compound of the general formula (III): 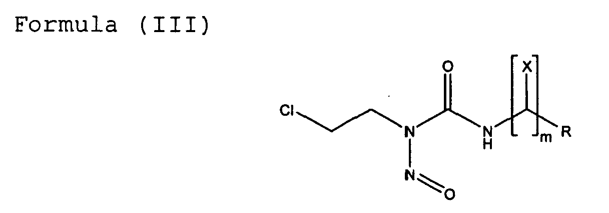 wherein
R is selected from the group consisting of halogen, alkyl, cycloalkyl, aryl, heteroary which alkyl, cycloalkyl, aryl, and heteroaryl are optionally substituted at one or more positions with substituents selected from the group consisting of halogen, alkyl, cycloalkyl, haloalkyl, amino, alkylamino, hydroxy, alkoxy, haloalkoxy, aryl, and heterorayl, and
X is selected from the group consisting of hydrogen, alkyl, aryl, halogen, heteroaryl, phosphonic acid ester, hydroxy, amino, alkylamino, acylamino, alkoxy, haloalkoxy and C(O)OR', wherein R' is alkyl or haloalkyl, and
m is 0 or 1.

11. The use according to any one of claims 1 to 6, and 10 wherein the compound is selected from the group consisting of carmustine, fotemustine, lomustine, and nimustine.

12. The use according to any one of claims 1 to 4 wherein the cytotoxic and/or cytostatic compound or the prodrug thereof is a substrate of P-glycoprotein.

13. The use according to any one of claims 1 to 4, and 12 wherein the cytotoxic and/or cytostatic compound or the prodrug thereof is administered in combination with an inhibitor of P-glycoprotein.

14. The use according to any one of claims 1 to 4, 12 and 13 wherein the cytotoxic and/or cytostatic compound is a taxane or structural derivative of a taxane.

15. The use according to any one of claims 1 to 4, and 12 to 14 wherein the cytotoxic and/or cytostatic compound is paclitaxel or docetaxel.

16. The use according to any one of claims 1 to 4, 12 and 13 wherein the structure of the cytotoxic and/or cytostatic compound is **characterized by** at least four fused cyclic moieties, wherein the cyclic moieties are independently a saturated or non-saturated cycloalkyl or heterocycloalkyl, aryl or heteroaryl ring system.

17. The use according to any one of claims 1 to 4, 12, 13, and 16 wherein the cytotoxic and/or cytostatic compound is a vinca alkaloid.

18. The use according to any one of claims 1 to 4, 12, 13, 16 and 17 wherein the cytotoxic and/or cytostatic compound is selected from the group consisting of eburnamonine, vinblastine, vindesine, vincristine and vinorelbine.

19. The use according to any one of claims 1 to 4, 12, 13, and 16 wherein the cytotoxic and/or cytostatic compound is an anthracycline.

20. The use according to any one of claims 1 to 4, 12, 13, 16 and 19 wherein the cytotoxic and/or cytostatic compound is selected from the group consisting of doxorubicin, daunorubicine, idarubicine and epirubicine.

21. The use according to any one of claims 13 to 20 wherein the inhibitor of P-glycoprotein is selected from the group consisting of verapamil, dexverapamil, nifedipine, dexniguldipine, amiodarone, bepridil, cyclosporin A, valspodar, cinchonine, quinine, quinidine, tamoxifen, toremifene, progesterone, dipyridamole, trifluoperazine, trans-flupentixol, biricodar (VX-710), S-9788, BIBW-22, and laniquidar (R-101933).

22. The use according to any one of claims 13 to 20 wherein the inhibitor of P-glycoprotein is a compound of the general formula (IV) or a prodrug thereof: 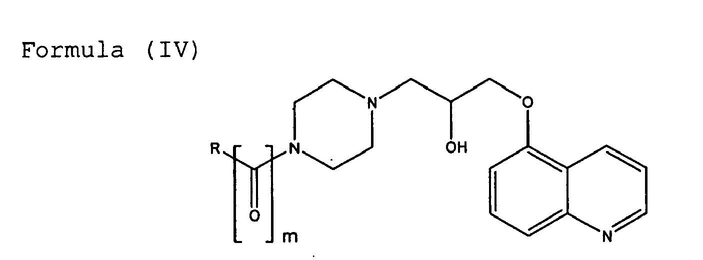 wherein
m is 0 or 1, and
R is independently selected from the group consisting of aryl, heteroaryl, cycloalkyl optionally containing 1 or 2 heteroatoms, and 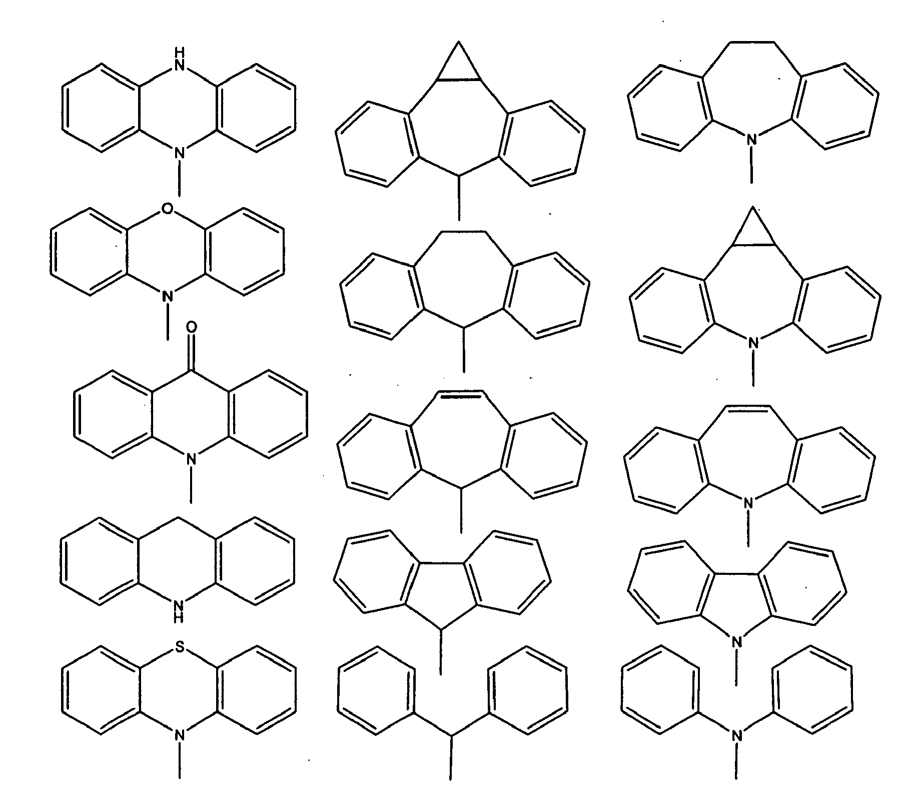 which groups are optionally substituted at one or more positions with substituents selected from the group consisting of halogen, alkyl, haloalkyl, hydroxy, alkoxy, haloalkoxy, amino, alkylamino, carboxyl, and acylamino.

23. The use according to any one of claims 13 to 20, and 22 wherein the P-glycoprotein inhibitor is zosuquidar (LY-335979) or MS-209.

24. The use according to any one of claims 13 to 20 wherein the inhibitor of P-glycoprotein is a compound of the general formula (V) or a prodrug thereof: 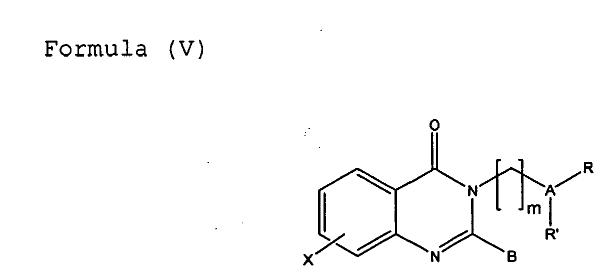 wherein
X is selected from the group consisting of hydrogen, halogen, alkyl, haloalkyl, hydroxy, alkoxy, haloalkoxy, amino, and alkylamino,
m is 0, 1 or 2,
A is selected from the group consisting of aryl, heteroaryl, cycloalkyl which aryl, heteroaryl and cycloalkyl are optionally substituted at one ore more positions with substituents selected from the group consisting of halogen, alkyl, amino, alkylamino, hydroxy, alkoxy, haloaryl, and haloalkoxy,
B is a carbocyclic or heterocyclic, aromatic or nonaromatic moiety optionally substituted at one or more positions with substituents selected from the group consisting of halogen, alkyl, haloalkyl, hydroxy, alkoxy, haloalkoxy, amino, and alkylamino,
R and R' are independently selected from the group consisting of hydrogen, alkyl, alkoxy, aryl, heteroaryl, benzyl, benzoyl, alkoxy, haloalkoxy, halogen and hydroxy.

25. The use according to any one of claims 13 to 20 wherein the inhibitor of P-glycoprotein is a compound of the general formula (VI) or a prodrug thereof: 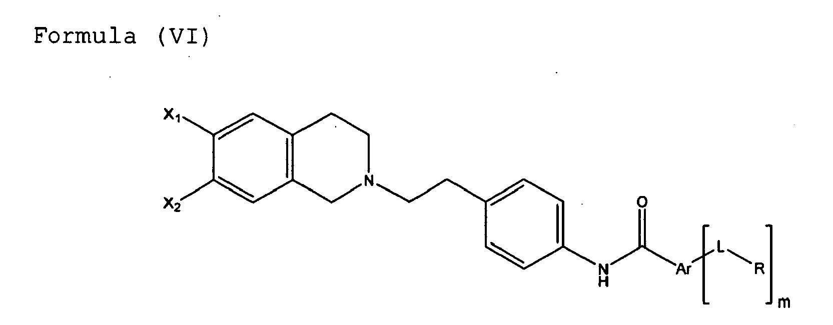 wherein
X₁ and X₂ are independently selected from the group consisting of hydrogen, halogen, alkyl, haloalkyl, hydroxy, alkoxy, haloalkoxy, amino, and alkylamino,
Ar is aryl, heteroaryl or 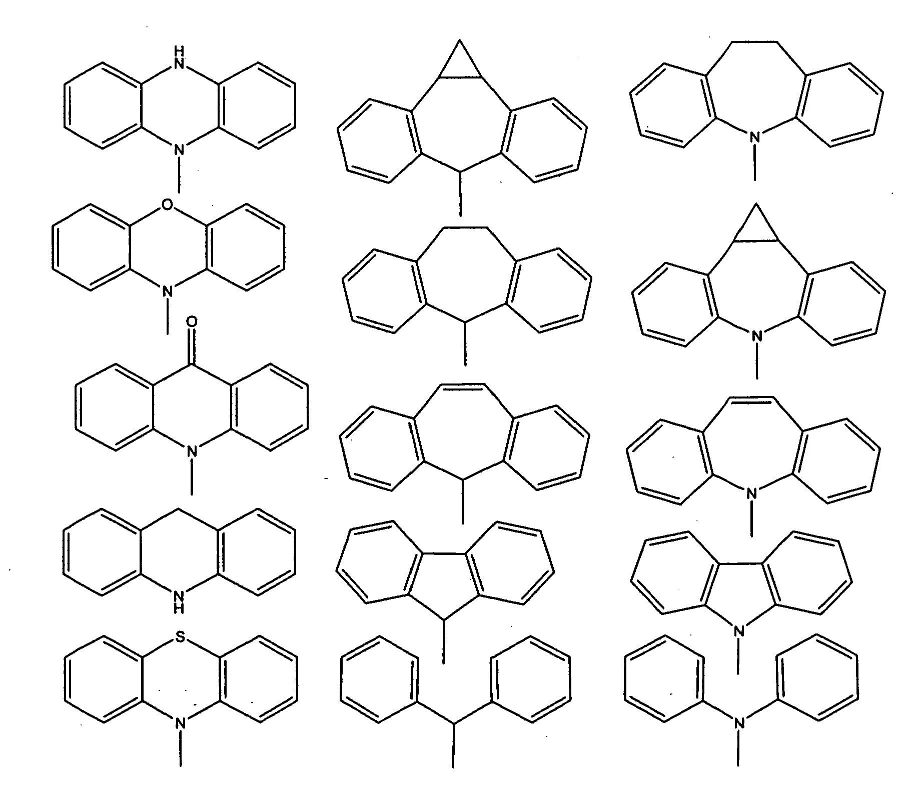 optionally substituted at one ore more positions with substituents selected from the group consisting of halogen, alkyl, haloalkyl, hydroxy, alkoxy, haloalkoxy, amino, and alkylamino,
L is selected from the group consisting of: 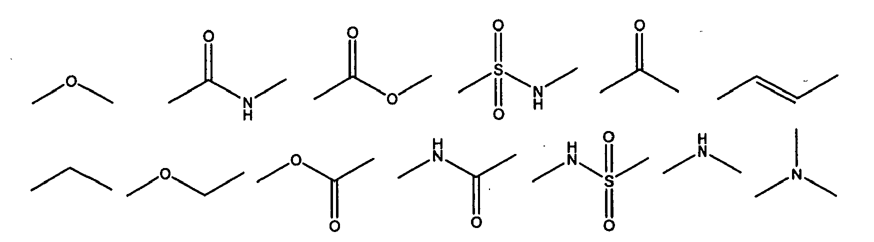
R is selected from the group consisting of alkyl, aryl, and heteroaryl, and
m is 0 or 1.

26. The use according to any one of claims 13 to 20, and 25 wherein the inhibitor of P-glycoprotein is tariquidar (XR-9576) or elacridar (GF-120918).

27. The use according to any one of claims 13 to 20 wherein the inhibitor of P-glycoprotein is a modified or unmodified cyclic peptide.

28. The use according to claim 27 wherein the cyclic peptide comprises 8 - 12 amino acids.

29. The use according to any one of claims 13 to 20, 27 and 28 wherein the inhibitor of P-glycoprotein is cyclosporin A or valspodar.

30. The use according to any one of claims 1 to 29 wherein an established treatment of a protozoal disease is co-administered with the pharmaceutical composition.
